# EUROPEAN PATENT SPECIFICATION

(11) **EP 1 545 590 B1**
(45) Date of publication and mention of the grant of the patent: **21.06.2006**
(21) Application number: 02785892.7
(22) Date of filing: 01.10.2002
(51) Int. Cl.: A61K 38/54, A61P 27/02

(54) **ENZYMATIC TREATMENT OF RETINITIS PIGMENTOSA AND RELEVANT PHARMACEUTICAL COMPOSITION IN FORM OF A KIT**
ENZYMATISCHE BEHANDLUNG VON RETINITIS PIGMENTOSA UND PHARMAZEUTISCHE ZUSAMMENSETZUNG HIERFÜR IN FORM EINES KITS
TRAITEMENT ENZYMATIQUE DE RETINITIS PIGMENTOSA ET COMPOSITION PHARMACEUTIQUE SOUS FORME DE KIT

(43) Date of publication of application: 29.06.2005
(73) Proprietor: Ammannati, Paola, 56125 Pisa (IT); Giordani, Roberto, 56125 Pisa (IT)
(72) Inventor: Ammannati, Paola, 56125 Pisa (IT); Giordani, Roberto, 56125 Pisa (IT)
(74) Representative: Bardini, Marco Luigi
(86) International application number: PCT/IT2002/000624
(87) International publication number: WO 2004/030693

(56) References cited:
- FR-A- 2 784 030
- FR-A- 2 784 898
- ZENG L.H.; WU D.Z.; MA Q.Y.; WU L.: "Genetic Study of Retinitis Pigmentosa in China" OPHTHALMOLOGICA, vol. 194, no. 1, 1987, pages 34-39, XP009010425
- GLORIA E.M.; AMMANNATI P.; SIRAVO D.: "The Pathogenesis of Retinitis Pigmentosa. A Pilot Study on the Clinical Fluoroangiographic and enzymatic effect of Bendazac Lysine" BOLLETTINO DI OCULISTICA, vol. 69, no. 2, 1990, pages 309-318, XP002240485

## Description

### Field of the invention

The present invention concerns a treatment of retinitis pigmentosa by means of enzymes and a pharmaceutical composition in kit form that can be used for this treatment.

### Description of the prior art

Retinitis pigmentosa is a disease of the retina that presents many different pathological manifestations: it may bring about a restriction of the field of vision and create increasing difficulty in adapting to the dark and to penumbra, when it affects the peripheral zones of the retina, which accommodate the greater part of the rod cells that render possible vision in penumbra and the perception of movement in the lateral zones, or may lead to loss of central vision when the cone cells are the ones to undergo modification. The rate of progress of the illness varies from one subject to another. As a general rule, retinitis pigmentosa manifests itself in youth, but often also affects children and acts in an insidious manner.

The causes responsible for this infirmity are as yet unknown and, consequently, there does not exist any cure for those affected by it. The sole certain information at the disposal of experts regards the genetic origin of retinitis pigmentosa, which is in part transmitted by heredity from generation to generation, following mechanisms that are known to geneticists. The greater part of the forms of retinitis pigmentosa are hereditary and three transmission modalities have so far been identified: dominantly autosomal, recessively autosomal and bound up with sex (X=linked).

The principal symptoms of the illness are crepuscular and nocturnal blindness, i.e. difficulty of seeing when the lighting conditions are poor, and problems of adaptation from well-lit to dark environments or vice versa. This phenomenon is due to the fact that, at least in the greater part of cases, attack in the early development phases of the illness is concentrated on the rod cells. Other typical symptoms are the reaction to excessively strong light (dazzlement), a gradual narrowing of the visual field, which manifests itself in the form of difficulty in perceiving objects situated on either side or stumbling over steps or other low obstacles, eventually arriving at complete blindness.

The course of the illness is of extremely variable duration, but is always gradual and ultimately leads to disablement. In the greater part of cases the previously described symptoms become aggravated, the visual field becomes more and more restricted and eventually closes completely. Other complaints tend to appear, among them dazzlement, incapacity of distinguishing colours and a particular form of cataract. In many cases the final outcome is, unfortunately, absolute blindness.

For the purpose of diagnosing the illness it is usual to rely on such tests as examination of the fundus of the eye, examination of the visual field, electroretinograms, fluorangiography, and visus examination:
- examination of the fundus of the eye aims at assessing the condition of the retina and to look for the presence of the characteristic pigment spots on the retinal surface, which in the illness assume a characteristic "osteoblast-like" appearance. Though they present the same symptoms, some rare forms of retinitis pigmentosa are not however characterized by spots on the fundus of the eye;
- examination of the visual field makes it possible to evaluate the sensitivity of the various parts of the retina to light stimuli. It will be useful to have an objective documentation of the difficulty in visual perception experienced by the patient;
- the electroretinogram (ERG) consists of recording the electrical activity of the retina in response to particular light,stimuli, thus making possible distinct valuations of the functionality of the two different types of photoreceptors (i.e. cone cells and rod cells). The electroretinogram is a very important examination for diagnosing retinitis pigmentosa, because - even when the illness is in its initial stages - the resulting trace is almost always either very flat or altogether absent;
- fluorangiography is performed by means of the intravenous injection of a fluorescent substance and subsequent photography of the retina at different times. Due to blood circulation, in fact, the fluorescent substance arrives at the retina, where it colours the arteries, the capillaries and the veins and thus renders them visible, as also the functional state of their walls;
- visus examination permits a valuation of visual acuity and consists of the patient reading letters of different sizes at a distance of three metres.

Although retinitis pigmentosa was identified and classified about midway through last century, very little concrete progress has so far been achieved either on the front of possible cures or on the equally important front of understanding the causes that determine and regulate its course. The lines at present most widely followed by international research are the genetic approach, which seeks to identify the gene or genes responsible for the illness and thus permitting a subsequent intervention by means of modern genetic engineering techniques, the transplant approach, which aims at perfecting a technique that would make possible the transplant of retinal tissue or, at least, the grafting of healthy cells into diseased retinas, and the immunological approach, which sets out to verify some theories that assume the illness to be underlain by some alteration of the immunological system.

The object of the present invention is to provide a pharmacological composition in kit form that will permit retinitis pigmentosa to be efficiently treated, so as to obtain a gradual recovery of visual acuity and enlargement of the field of vision, the sharpness of images and the perception of colours and the reconstitution of a normal electroretinogram in the long run.

According to the present invention, this aim is attained by means of the use of particular enzymes for incorporation in a pharmaceutical composition in kit form to be employed for treating retinitis pigmentosa by means of injection into the retrobulbar tissue, all as specified in Claim 1.

More particularly, the enzymes employed are glutathione peroxidase (hereinafter referred to as Enzyme A), prolidase (hereinafter referred to as Enzyme B), glucose-6-phosphate-dehydrogenase (hereinafter referred to as Enzyme C), which are administered in accordance with a time sequence and modalities to be further specified hereinbelow.

Optionally the treatment may also provide the use of aldose reductase (hereinafter referred to as Enzyme D), which has been found to be useful when - as happens in the greater part of cases - the patient complains of visual fogging.

The enzymes employed in the treatment in accordance with the present invention are available in commerce in lyophilized form and are dissolved in physiological solution to render them available for the treatment.

Each enzyme - in the form of enzyme solution - is administered by means of retrobulbar injection into each eye for three consecutive days, repeating the administration on another two occasions, each separated from its predecessor by a period of one month (for each enzyme). In practice the procedure is as follows:
- one dose of Enzyme A is injected into the retrobulbar tissue of each eye for three consecutive days at the beginning of the treatment, the treatment being then repeated in the second and third month;
- in the fourth, fifth and sixth month one dose of Enzyme B is injected into the retrobulbar tissue of each eye for three consecutive days;
- in the seventh, eighth and ninth month one dose of Enzyme C is injected into the retrobulbar tissue of each eye for three consecutive days;
- if necessary, in the next three months and for three days in each month the treatment is then continued with Enzyme D, the administration mode being exactly as before.

The doses of the various enzymes used at each injection (for each eye) are as follows:

| | |
|---|---|
| Enzyme A | 0.03 - 0.05 U.I. |
| Enzyme B | 5 - 7 U.I. |
| Enzyme C | 7 - 9 U.I. |
| Enzyme D | 7 - 9 U.I. |

The preferred doses of the various enzymes at each injection are as follows:

| | |
|---|---|
| Enzyme A | 0.04 U.I. |
| Enzyme B | 6.67 U.I. |
| Enzyme C | 8.00 U.I. |
| Enzyme D | 8.00 U.I. |

These doses remain the same for all patients, quite independently of the typology of the alteration. In particular, the enzyme solutions are prepared in such a way that the quantities set out above may be contained within an injection of 0.4 ml. For example, the enzyme solutions suitable for providing injectable doses of 0.4 ml containing the preferred enzyme quantities as set out above are to be prepared as follows
Enzyme A:
   Phial containing 10 U.I. of lyophilised enzyme, bring up to 100 ml with physiological solution.
Enzyme B:
   Phial containing 1000 U.I. of lyophilised enzyme, bring up to 60 ml with physiological solution.
Enzyme C:
   Phial containing 2000 U.I. of lyophilised enzyme, bring up to 100 ml with physiological solution.
Enzyme D:
   Phial containing 2000 U.I. of lyophilised enzyme, bring up to 100 ml with physiological solution.

Naturally, these ratios will have to be modified if it is decided to change the dose of any one of the enzymes within its dosing range as set out above.

The enzymes are administered in the order in which they are stated above and the injection cycles are to be continued without interruption.

The kit used for treating retinitis pigmentosa in accordance with the present invention contains the aforementioned enzymes in aliquot parts and interactive quantities appropriate for administering:
a) Enzyme A at a concentration comprised between 0.03 and 0.05 U.I. in 0.4 ml of physiological solution for three consecutive days, at monthly intervals, for three consecutive days and for each eye;
b) Enzyme B, starting from the month following the last administration of Enzyme A, at a concentration of 5 to 7 U.I. in 0.4 ml of physiological solution for three consecutive days, at monthly intervals, for three months and for each eye;
c) Enzyme C, starting from the month following the last administration of Enzyme B, at a concentration of 7 to 9 U.I. in 0.4 ml of physiological solution for three consecutive days, at monthly intervals, for three months and for each eye.

Optionally, the kit may also contain Enzyme D, to be administered, starting from the month following the last administration of Enzyme C, at a concentration of 7 to 9 U.I. in 0.4 ml of physiological solution for three consecutive days, at monthly intervals, for three months and for each eye.

In particular, the enzymes are contained in each kit in lyophilized form and in quantities sufficient for at least one complete series of administrations, each enzyme being subdivided into aliquot parts containing a quantity sufficient for one three-month injection cycle, i.e. eighteen injections, or for one daily administration, i.e. two injections, and optionally the appropriate doses of physiological solution for constituting said aliquot parts. In particular, in each kit the various enzymes are subdivided into one or more aliquot parts, each of which contains, in the preferred dosing forms as set out above, from 0.04 U.I to 0.72 U.I of Enzyme A, from 6.67 U.I. to 120 U.I. of Enzyme B, from 8 U.I to 144 U.I of Enzyme C and possibly from 8 U.I to 144. U.I. of Enzyme D. Possibly there may also be present three or more aliquot parts of physiological solution from 0.4 to 7.2 ml each.

Patients subjected to the treatment in accordance with the invention found a gradual improvement of visual acuity and field of vision, colour perception and image sharpness (definition). Their electroretinograms improved little by little, eventually becoming reconstituted in the long run. The administered treatment produced a positive response in all the patients, albeit over different periods of time. Follow-up checks after 5-8 years showed that the obtained improvement was permanent and did not bring out side effects of any kind.

Some experimental data confirm the hypothesis that retinitis pigmentosa could be due to an enzyme defect that alters the metabolism of the retina, modifying not only the vision process, but facilitating also the accumulation of pigments, the characteristic feature of the illness.

Studies carried out on rabbits (New Zealand/Fulvo di Borgogna) by one of the applicants showed that the total inhibition of some enzymes causes variations of the electroretinogram, with reduction of the depolarisation wave to the point of extinction and reconstitution of the normal trace after retrobulbar administration of glutathione peroxidase (Enzyme A) and prolidase (Enzyme B). Erythrocyte level reduction of glucose-6-phosphate dehydrogenase and glutathione peroxidase in patients affected by retinitis pigmentosa was subsequently demonstrated by means of a comparison of their enzyme erythrocyte concentrations with those of subjects not affected by this illness, seemingly in good health, the two groups being homogeneous as regards sex and age.

## Claims

1. A pharmaceutical kit for the treatment of retinitis pigmentosa containing the enzymes glutathione peroxidase (Enzyme A), prolidase (Enzyme B), glucose-6-phosphate dehydrogenase (Enzyme C) and, optionally, aldose reductase (Enzyme D) in aliquot parts and interactive quantities appropriate for administering:
a) Enzyme A at a concentration comprised between 0.03 and 0.05 U.I. in 0.4 ml of physiological solution for three consecutive days, at monthly intervals, for three months and for each eye;
b) Enzyme B, starting from the month following the last administration of Enzyme A, at a concentration of 5 to 7 U.I. in 0.4 ml of physiological solution for three consecutive days, at monthly intervals, for three months and for each eye;
c) Enzyme C, starting from the month following the last administration of Enzyme B, at a concentration of 7 to 9 U.I. in 0.4 ml of physiological solution for three consecutive days, at monthly intervals, for three months and for each eye.
d) Enzyme D, starting from the month following the last administration of Enzyme C, at a concentration of 7 to 9 U.I. in 0.4 ml of physiological solution for three consecutive days, at monthly intervals, for three months and for each eye.

2. A kit in accordance with Claim 1, wherein the concentration of Enzyme A is 0.04 U.I in 0.4 ml of physiological solution, the concentration of Enzyme B is 6.67 U.I. in 0.4 ml of physiological solution and the concentration of Enzyme C is 8 U.I in 0.4 ml of physiological solution, the concentration of optional Enzyme D being equal to 8 U.I. in 0.4 ml of physiological solution.

3. A kit in accordance with Claim 1 or Claim 2, wherein said kit comprises said enzymes in lyophilised form, in quantities sufficient for at least one series of administrations of from a) to c) and, optionally, also d), subdivided into aliquot parts containing - for each enzyme - a quantity of enzyme sufficient for the constitution of said aliquot parts.

4. A kit in accordance with any one of Claims 1 to 3, wherein said kit comprises said enzymes in lyophilised form subdivided into one or more aliquot parts, each containing from 0.04 U.I. to 0.72 U.I. of Enzyme A, from 0.67 to 120 U.I of Enzyme B, from 8 to 144 U.I of Enzyme C and, optionally, from 8 to 144 U.I of Enzyme D, and, optionally, three or more aliquot parts of physiological solution of from 0.4 to 7.2 ml each.

5. Use of the enzymes glutathione peroxidase (Enzyme A), prolidase (Enzyme B), glucose-6-phosphate dehydrogenase (Enzyme C) and, optionally, aldose reductase (Enzyme D) for the preparation of a pharmaceutical composition in kit form for the treatment of retinitis pigmentosa by means of injection into the retrobulbar tissue, said kit containing said enzymes in aliquot parts and interactive quantities appropriate for administering:
a) Enzyme A at a concentration comprised between 0.03 and 0.05 U.I. in 0.4 ml of physiological solution for three consecutive days, at inonthly intervals, for three months and for each eye;
b) Enzyme B, starting from the month following the last administration of Enzyme A, at a concentration of 5 to 7 U.I. in 0.4 ml of physiological solution for three consecutive days, at monthly intervals, for three months and for each eye;
c) Enzyme C, starting from the month following the last administration of Enzyme B, at a concentration of 7 to 9 U.I. in 0.4 ml of physiological solution for three consecutive days, at monthly intervals, for three months and for each eye.
d) Enzyme D, starting from the month following the last administration of Enzyme C, at a concentration of 7 to 9 U.I. in 0.4 ml of physiological solution for three consecutive days, at monthly intervals, for three months and for each eye.

6. Use of the enzymes in accordance with Claim 5, wherein concentration of Enzyme A is 0.04 U.I in 0.4 ml of physiological solution, the concentration of Enzyme B is 6.67 U.I. in 0.4 ml of physiological solution and the concentration of Enzyme C is 8 U.I in 0.4 ml of physiological solution, the concentration of optional Enzyme D being equal to 8 U.I. in 0.4 ml of phisiological solution.

7. Use of the enzymes in accordance with Claims 5 or 6, wherein said kit comprises said enzymes in lyophilised form, in quantities sufficient for at least one series of administrations of from a) to c) and, optionally, also d), subdivided into aliquot parts containing - for each enzyme - a quantity of enzyme sufficient for the constitution of said aliquot parts.

8. Use of the enzymes in accordance with claims 5-7, wherein said kit comprises said enzymes in lyophilised form subdivided into one or more aliquot parts, each containing from 0.04 U.I. to 0.72 U.I of Enzyme A, from 0.67 to 120 U.I of Enzyme B, from 8 to 144 U.I of Enzyme C and, optionally, from 8 to 144 U.I of Enzyme D, and, optionally, three or more aliquot parts of physiological solution of from 0.4 to 7.2 ml each.

## Patentansprüche

1. Pharmazeutisches Kit für die Behandlung von Retinitis Pigmentosa, enthaltend die Enzyme Glutathion-Peroxidase (Enzym A), Prolidase (Enzym B), Glukose-6-Phosphat-Dehydrogenase (Enzym C) und optional Aldose-Reduktase (Enzym D) in aliquoten Teilen und interaktiven Mengen, geeignet zur Verabreichung von:
a) Enzym A mit einer Konzentration, die zwischen 0,03 und 0,05 U.I. in 0,4 ml einer physiologischen Lösung enthalten ist, für drei aufeinander folgende Tage in monatlichen Intervallen für drei Monate und für jedes Auge;
b) Enzym B beginnend von dem Monat an, das der letzten Verabreichung von Enzym A folgt, mit einer Konzentration von 5 bis 7 U.I. in 0,4 ml einer physiologischen Lösung, für drei aufeinander folgende Tage in monatlichen Intervallen für drei Monate und für jedes Auge;
c) Enzym C beginnend von dem Monat an, das der letzten Verabreichung von Enzym B folgt, mit einer Konzentration von 7 bis 9 U.I. in 0,4 ml einer physiologischen Lösung, für drei aufeinander folgende Tage in monatlichen Intervallen für drei Monate und für jedes Auge;
d) Enzym D beginnend von dem Monat an, das auf die letzte Verabreichung von Enzym C folgt, mit einer Konzentration von 7 bis 9 U.I. in 0,4 ml einer physiologischen Lösung, für drei aufeinander folgende Tage in monatlichen Intervallen für drei Monate und für jedes Auge.

2. Kit nach Anspruch 1, wobei die Konzentration von Enzym A 0,04 U.I. in 0,4 ml einer physiologischen Lösung ist, die Konzentration von Enzym B 6,67 U.I. in 0,4 ml einer physiologischen Lösung ist, und die Konzentration von Enzym C 8 U.I. in 0,4 ml einer physiologischen Lösung ist, wobei die Konzentration von optionalem Enzym D gleich 8 U.I. in 0,4 ml einer physiologischen Lösung ist.

3. Kit nach Anspruch 1 oder Anspruch 2, wobei das Kit die Enzyme in lyophilisierter Form in Mengen enthält, die für wenigstens eine Serie von Verabreichungen von a) bis c) und optional auch d) ausreichend sind, unterteilt in aliquote Teile, die - für jedes Enzym - eine Enzymmenge enthalten, die für die Bildung der aliquoten Teile ausreichend ist.

4. Kit nach einem der Ansprüche 1 bis 3, wobei das Kit die Enzyme in lyophilisierter Form in ein oder mehrere aliquote Teile unterteilt enthält, von welchen jeder von 0,04 U.I. bis 0,72 U.I. des Enzymes A, von 0,67 bis 120 U.I. des Enzymes B, von 8 bis 144 U.I. des Enzymes C und optional von 8 bis 144 U.I. des Enzymes D und optional drei oder mehrere aliquote Teile einer physiologischen Lösung von jeweils 0,4 bis 7,2 ml enthält.

5. Verwendung der Enzyme Glutathion-Peroxidase (Enzym A), Prolidase (Enzym B), Glukose-6-Phosphat-Dehydrogenase (Enzym C) und optional Aldose-Reduktase (Enzym D) für die Präparation einer pharmazeutischen Zusammensetzung in einer Kit-Form für die Behandlung von Retinitis Pigmentosa mittels Injektion in das retrobulbare Gewebe, welches Kit die Enzyme in aliquoten Teilen und interaktiven Mengen enthält, geeignet zur Verabreichung von:
a) Enzym A mit einer Konzentration, die zwischen 0,03 und 0,05 U.I. in 0,4 ml einer physiologischen Lösung enthalten ist, für drei aufeinander folgende Tage in monatlichen Intervallen für drei Monate und für jedes Auge;
b) Enzym B beginnend von dem Monat an, das der letzten Verabreichung von Enzym A folgt, mit einer Konzentration von 5 bis 7 U.I. in 0,4 ml einer physiologischen Lösung, für drei aufeinander folgende Tage in monatlichen Intervallen für drei Monate und für jedes Auge;
c) Enzym C beginnend von dem Monat an, das der letzten Verabreichung von Enzym B folgt, mit einer Konzentration von 7 bis 9 U.I. in 0,4 ml einer physiologischen Lösung, für drei aufeinander folgende Tage in monatlichen Intervallen für drei Monate und für jedes Auge;
d) Enzym D beginnend von dem Monat an, das auf die letzte Verabreichung von Enzym C folgt, mit einer Konzentration von 7 bis 9 U.I. in 0,4 ml einer physiologischen Lösung, für drei aufeinander folgende Tage in monatlichen Intervallen für drei Monate und für jedes Auge.

6. Verwendung der Enzyme gemäß Anspruch 5, wobei die Konzentration von Enzym A 0,04 U.I. in 0,4 ml einer physiologischen Lösung ist, die Konzentration von Enzym B 6,67 U.I. in 0,4 ml einer physiologischen Lösung ist, und die Konzentration von Enzym C 8 U.I. in 0,4 ml einer physiologischen Lösung ist, wobei die Konzentration von optionalem Enzym D gleich 8 U.I. in 0,4 ml einer physiologischen Lösung ist.

7. Verwendung der Enzyme gemäß Anspruch 5 oder 6, wobei das Kit die Enzyme in lyophilisierter Form in Mengen enthält, die für wenigstens eine Serie von Verabreichungen von a) bis c) und optional auch d) ausreichend sind, unterteilt in aliquote Teile, die - für jedes Enzym - eine Enzymmenge enthalten, die für die Bildung der aliquoten Teile ausreichend ist.

8. Verwendung der Enzyme gemäß den Ansprüchen 5 - 7, wobei das Kit die Enzyme in lyophilisierter Form in ein oder mehrere aliquote Teile unterteilt enthält, von welchen jeder von 0,04 U.I. bis 0,72 U.I. des Enzymes A, von 0,67 bis 120 U.I. des Enzymes B, von 8 bis 144 U.I. des Enzymes C und optional von 8 bis 144 U.I. des Enzymes D und optional drei oder mehrere aliquote Teile einer physiologischen Lösung von jeweils 0,4 bis 7,2 ml enthält.

## Revendications

1. Un kit pharmaceutique pour le traitement de la rétinite pigmentaire contenant les enzymes peroxydase de glutathione (Enzyme A), prolidase (Enzyme B), déhydrogénase de glucose-6-phosphate (Enzyme C) et, optionnellement, aldose réductase (Enzyme D) en parties aliquotes et en quantités interactives appropriées pour l'administration :
a) Enzyme A à une concentration comprise entre 0,03 et 0,05 U.I. dans 0,4 ml de solution physiologique pendant trois jours consécutifs, à intervalles mensuels, pendant trois mois et pour chaque oeil ;
b) Enzyme B, à partir du mois suivant la dernière administration de l'Enzyme A, à une concentration de 5 à 7 U.I. dans 0,4 ml de solution physiologique pendant trois jours consécutifs, à intervalles mensuels, pendant trois mois et pour chaque oeil;
c) Enzyme C, à partir du mois suivant la dernière administration de l'Enzyme B, à une concentration de 7 à 9 U.I. dans 0,4 ml de solution physiologique pendant trois jours consécutifs, à intervalles mensuels, pendant trois mois et pour chaque oeil;
d) Enzyme D, à partir du mois suivant la dernière administration de l'Enzyme C, à une concentration de 7 à 9 U.I. dans 0,4 ml de solution physiologique pendant trois jours consécutifs, à intervalles mensuels, pendant trois mois et pour chaque oeil.

2. Un kit en accord avec la revendication 1, dans lequel la concentration de l'Enzyme A est de 0,04 U. I. dans 0,4 ml de solution physiologique, la concentration de l'Enzyme B est de 6,67 U. 1. dans 0,4 ml de solution physiologique et la concentration de l'Enzyme C est de 8 U.I. dans 0,4 ml de solution physiologique, la concentration de l'Enzyme optionnelle D étant égale à 8 U.I. dans 0,4 ml de solution physiologique.

3. Un kit en accord avec la revendication 1 ou la revendication 2, dans lequel ledit kit comprend lesdites enzymes sous forme lyophilisée, en quantités suffisantes pour au moins une série d'administrations de a) à c) et, optionnellement, aussi d), divisées en parties aliquotes contenant - pour chaque enzyme - une quantité d'enzyme suffisante pour la constitution des dites parties aliquotes.

4. Un kit en accord avec l'une quelconque de revendications 1 à 3, dans lequel ledit kit comprend lesdites enzymes sous forme lyophilisée divisées en une ou plusieurs parties aliquotes, chacune contenant de 0,04 U.I. à 0,72 U.I. d'Enzyme A, de 0,67 à 120 U.I. d'Enzyme B, de 8 à 144 U.I. d'Enzyme C et, optionnellement, de 8 à 144 U.I. d'Enzyme D, et, optionnellement, trois ou plus parties aliquotes de solution physiologique de 0,4 à 7,2 ml chacune.

5. Utilisation des enzymes peroxydase de glutathione (Enzyme A), prolidase (Enzyme B), déhydrogénase glucose-6-phosphate (Enzyme C) et, optionnellement, aldose réductase (Enzyme D) pour la préparation d'une composition pharmaceutique sous la forme de kit pour le traitement de la rétinite pigmentaire au moyen d'injections dans le tissu rétrobulbaire, ledit kit contenant lesdites enzymes en parties aliquotes et en quantités interactives appropriées pour l'administration :
a) Enzyme A à une concentration comprise entre 0,03 et 0,05 U.I. dans 0,4 ml de solution physiologique pendant trois jours consécutifs, à intervalles mensuels, pendant trois mois et pour chaque oeil;
b) Enzyme B, à partir du mois suivant la dernière administration de l'Enzyme A, à une concentration de 5 à 7 U.I. dans 0,4 ml de solution physiologique pendant trois jours consécutifs, à intervalles mensuels, pendant trois mois et pour chaque oeil;
c) Enzyme C, à partir du mois suivant la dernière administration de l'Enzyme B, à une concentration de 7 à 9 U.I. dans 0,4 ml de solution physiologique pendant trois jours consécutifs, à intervalles mensuels, pendant trois mois et pour chaque oeil;
d) Enzyme D, à partir du mois suivant la dernière administration de l'Enzyme C, à une concentration de 7 à 9 U.I. dans 0,4 ml de solution physiologique pendant trois jours consécutifs, à intervalles mensuels, pendant trois mois et pour chaque oeil.

6. Utilisation des enzymes en accord avec la revendication 5, dans laquelle la concentration de l'Enzyme A est de 0,04 U.I. dans 0,4 ml de solution physiologique, la concentration de l'Enzyme B est de 6,67 U.I. dans 0,4 ml de solution physiologique et la concentration de l'Enzyme C est de 8 U.I. dans 0,4 ml de solution physiologique, la concentration de l'Enzyme optionnelle D étant égale à 8 U.I. dans 0,4 ml de solution physiologique.

7. Utilisation des enzymes en accord les revendications 5 ou 6, dans laquelle ledit kit comprend lesdites enzymes sous forme lyophilisée, en quantités suffisantes pour au moins une série d'administrations de a) à c) et, optionnellement, aussi d), divisées en parties aliquotes contenant - pour chaque enzyme - une quantité d'enzyme suffisante pour la constitution des dites parties aliquotes.

8. Utilisation des enzymes en accord avec les revendications 5 à 7, dans laquelle ledit kit comprend lesdites enzymes sous forme lyophilisée divisées en une ou plusieurs parties aliquotes, chacune contenant de 0,04 U.I. à 0,72 U.I. d'Enzyme A, de 0,67 à 120 U.I. d'Enzyme B, de 8 à 144 U.I. d'Enzyme C et, optionnellement, de 8 à 144 U.I. d'Enzyme D, et, optionnellement, trois ou plus parties aliquotes de solution physiologique de 0,4 à 7,2 ml chacune.
